(19) Europäisches Patentamt / European Patent Office / Office européen des brevets

(11) **EP 3 714 773 A1**

(12) **DEMANDE DE BREVET EUROPEEN**

(43) Date de publication:
**30.09.2020   Bulletin 2020/40**

(51) Int Cl.:
*A61B 5/00* *(2006.01)*       *A43B 5/00* *(2006.01)*
*A61B 5/11* *(2006.01)*       *A61B 5/01* *(2006.01)*
*A61B 5/103* *(2006.01)*

(21) Numéro de dépôt: **20166526.2**

(22) Date de dépôt: **27.03.2020**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité:  **29.03.2019   FR 1903424**

(71) Demandeur: **Zhor Tech**
**54000 Nancy (FR)**

(72) Inventeur: **OUMNIA, Karim**
**54000 NANCY (FR)**

(74) Mandataire: **A.P.I. Conseil**
**Immeuble Newton**
**4, rue Jules Ferry**
**64000 Pau (FR)**

(54)    **SYSTÈME DE CARACTÉRISATION DE LA POSTURE ET DE LA DÉMARCHE**

(57)    L'invention porte sur un procédé et un système (1) de caractérisation de la posture et de la démarche d'un utilisateur, ledit système étant caractérisé en ce qu'il comporte :
Une paire de boitiers électroniques (100, 101, 102), aptes à être intégrés dans une paire (10) de semelles, chaque boitier comprenant : une plateforme inertielle (110, 111, 112), et un moyen de communication (140, 141, 142) ;

Un dispositif autonome embarqué (20) comprenant : un module de communication (21) et un module de stockage de données (22) ; et
Un dispositif d'analyse (30) configuré pour traiter l'ensemble de données recueillies en fonction d'algorithmes prédéfinis et générer une information sur la posture et la démarche d'un utilisateur de la paire de semelles.

FIG. 1

EP 3 714 773 A1

## Description

L'invention concerne le domaine de la chaussure ou plus généralement celui des articles chaussants. L'invention concerne plus particulièrement un système de caractérisation ou de qualification de la démarche et de la posture comportant deux boitiers électroniques pour semelles de chaussures connectées, un dispositif autonome embarqué et un dispositif d'analyse.

### [Art antérieur]

Les chaussures peuvent être à usage de détente, formel, sportif, médical, professionnel ou plus simplement récréatif. Une chaussure se compose principalement d'une semelle plus ou moins relevée à l'arrière par le talon d'une part, et de la tige, partie supérieure qui enveloppe le pied, d'autre part.

Les semelles, qu'elles soient intérieures ou extérieures ont essentiellement pour rôle original de protéger le pied du sol. La semelle, partie inférieure d'une chaussure qui protège la plante des pieds, peut se décliner en deux parties. Une couche de semelle supérieure en contact direct avec le pied de l'utilisateur et une couche de semelle inférieure en contact direct avec le sol ou plus généralement l'environnement extérieur. Néanmoins, depuis plusieurs années maintenant, les nouvelles technologies de l'information accompagnent des besoins nouveaux et le monde de la chaussure fait partie de ce mouvement. Le développement de l'électronique a conduit à l'apparition de semelles et chaussures dites connectées, qui présentent des fonctions très diversifiées.

Parmi les semelles ou chaussures connectées, différents systèmes existent en vue de réaliser une pluralité de fonctions. Certains systèmes sont mis en oeuvre grâce à une seule chaussure ou une seule semelle connectée. Par exemple, le document EP1970671 et le document WO2011157870 décrivent chacun une chaussure intelligente conçue pour permettre à son utilisateur de commander différentes variables (distances parcourues, temps utilisé, calories consommées...) en vue de contrôler et améliorer ses performances sportives. Ce dispositif, basé sur un boitier unique, utilise un accéléromètre uniquement et pas de gyroscope, et ne permet de détecter ni la démarche ni la posture de son utilisateur. Ainsi, ces systèmes ne permettent pas d'offrir une analyse fine de la démarche d'un utilisateur, ou de générer des données de qualités.

D'autres systèmes ont été proposés, mettant en oeuvre des capteurs positionnés sur deux chaussures ou deux semelles d'une même paire connectées. Dans ces systèmes, les capteurs sont souvent dispersés dans les chaussures ou les semelles ce qui d'une part altère le confort de l'utilisateur et d'autre part, entraine des surcoûts importants associés à la fabrication de l'article chaussant. Par exemple, le document US2017/0241797 décrit un appareil ayant pour but chez l'utilisateur de compter les pas et de distinguer la course de la marche normale par le biais d'un podomètre et d'un accéléromètre. Ce dispositif n'offre cependant pas d'informations sur des paramètres autres que la vitesse. De plus, l'appareil ne contient qu'un seul podomètre et un seul accéléromètre ce qui ne permet pas la génération de valeurs paramètre avancés de démarche. Dans un autre exemple, le document US2017/0188950, propose des chaussures équipées de capteurs de pression, souvent peu fiables, et d'un accéléromètre et qui permettent de délivrer à un smartphone connecté via Bluetooth des statistiques sur les activités physiques de leur porteur tels que le nombre de pas réalisés ou la façon dont se positionne le pied durant la marche, etc. Le document US2017/303827 quant à lui décrit un dispositif de semelles connectées comportant des capteurs pour l'étude de la démarche d'une personne. Doté d'un accéléromètre et d'un gyroscope, ce dispositif recueille des données biomécaniques, qui sont échangées entre les deux semelles et transmises à un terminal. Toutefois, les différents éléments de ce dispositif sont disséminés dans la semelle, notamment les capteurs de pression qui recueillent l'essentiel des données.

Par ailleurs, ces dispositifs basés sur la présence de nombreux capteurs répartis dans la semelle (e.g. capteurs de pression) présentent une durée de vie plus faible et souvent une épaisseur relativement élevée pouvant limiter l'utilisation de ces semelles. En outre, les calculs ne sont généralement pas effectués en temps réel ce qui entraine des besoins élevés en termes de stockage de données et d'énergie.

Il existe des semelles utilisant des capteurs de pression censés identifier la posture, mais ces solutions techniques n'ont jamais permis à ce jour d'avoir un produit commercialisable et efficace.

Il existe donc un besoin pour un nouveau système équipé de deux chaussures ou semelles connectées permettant de rassembler des capteurs suffisants pour générer des données brutes de qualités afin d'analyser finement la démarche tout en étant compact, autonome et résistant.

### [Problème technique]

L'invention a pour but de remédier aux inconvénients de l'art antérieur. En particulier, l'invention a pour but de permettre à l'utilisateur d'accéder, particulièrement au travers du boitier électronique installé dans chacune de ses semelles connectées, à des informations sur la posture exacte de ses pieds et plus généralement sur sa démarche ou son activité.

En outre, l'invention a pour but de proposer une solution pour la caractérisation de la démarche qui soit compacte, résistante, d'autonomie élevée et qui soit de préférence configurée pour réaliser un traitement des données précis permettant une analyse fine de la démarche de l'utilisateur des chaussures connectées.

[Brève description de l'invention]

Ainsi, l'invention porte notamment sur un système de caractérisation de la posture et de la démarche d'un utilisateur, caractérisé en ce qu'il comporte :

- Une paire de boitiers électroniques, aptes à être intégrés dans une paire de semelles, un premier boitier étant apte à être intégré dans une première semelle et un second boitier étant apte à être intégré dans une seconde semelle, chaque boitier comprenant :

  ○ une plateforme inertielle configurée pour générer un ensemble de données sur un mouvement du pied d'un utilisateur de la paire de semelles,

  ○ un moyen de communication configuré de façon à transmettre l'ensemble généré de données à un dispositif autonome embarqué ; et

- un dispositif autonome embarqué comprenant :

  ○ un module de communication configuré pour recevoir les ensembles générés de données transmis par les deux boitiers électroniques, et

  ○ un module de stockage de données configuré pour mémoriser les ensembles générés de données réceptionnés,

  le module de communication étant en outre configuré pour transmettre les ensembles générés de données réceptionnés à un dispositif d'analyse ; et

- un dispositif d'analyse configuré pour traiter les ensembles de données recueillies en fonction d'algorithmes prédéfinis et générer une information sur la posture et la démarche d'un utilisateur de la paire de semelles.

Un tel système permet de suivre la démarche d'un utilisateur de façon fiable. En effet, la présence d'une paire de semelle comportant chacune un boitier protégeant une plateforme inertielle donne la possibilité de suivre de façon indépendante le mouvement de chacun des pieds. La plateforme inertielle va analyser, en au moins trois dimensions, la posture, les mouvements, la locomotion, l'équilibre et l'environnement de l'utilisateur, et plus généralement tout ce qui est lié à l'activité de ses pieds ou qui sera qualifié comme étant sa démarche. La plateforme inertielle va pouvoir non seulement constater les différentes positions mais également relever des carences ou des anomalies qui apparaissent au niveau de la locomotion, du pattern, ou plus généralement de la marche de l'utilisateur. En outre, le présent système est configuré de façon à ce que les boitiers électroniques transmettent régulièrement les données brutes générées par les plateformes inertielles ou prétraitées, au dispositif autonome embarqué. Ainsi, les boitiers électroniques pourront rester de taille réduite et présenter une autonomie élevée. Le dispositif autonome embarqué quant à lui transmet ces données au dispositif d'analyse qui disposera de toutes les ressources nécessaires pour une analyse fine de la démarche de l'utilisateur.

**Selon d'autres caractéristiques optionnelles du système :**

- les deux boitiers électroniques sont configurés pour communiquer entre eux et pour initier la génération de données sur le mouvement du pied d'un utilisateur seulement après réception d'un message du dispositif autonome embarqué et d'un message de l'autre boitiers électronique. Ainsi, les générations des données par les plateformes inertielles des deux boitiers sont synchronisées ce qui permet une analyse plus fine et plus juste de la démarche de l'utilisateur. Avantageusement, les boitiers électroniques sont configurés de façon à pouvoir vérifier ponctuellement leur synchronisation, de préférence pour vérifier leur synchronisation à une fréquence inférieure à 10 minutes.

- Il comporte au moins deux paires de boitiers électroniques, chacune configurée pour communiquer leurs ensembles générés de données au dispositif autonome embarqué. En outre, le dispositif autonome embarqué peut être configuré pour recevoir et retransmettre les ensembles de données générées par plusieurs paires de boitiers électroniques. En effet, le dispositif autonome embarqué permet de gérer plusieurs paires de boitiers électroniques et permet donc de concourir au suivi en simultané ou non et quasiment en temps réel la démarche de plusieurs utilisateurs. Dans cette configuration, le dispositif autonome embarqué est configuré pour transmettre tous les ensembles générés de données au dispositif d'analyse qui procédera au traitement conjoint des ensembles de données provenant de la même paire de semelle. Le dispositif autonome embarqué peut alors disposer d'identifiants et de codes nécessaires pour établir une communication avec chacune des paires de boitiers électroniques concernés.

- les ensembles générés de données sont cryptées par les boitiers électroniques et seul le dispositif d'analyse est apte à décrypter les données générées. Ainsi, les données générées sont protégées d'éventuelles attaques de tiers sur le dispositif autonome embarqué ou les boitiers électroniques. En effet, ni l'un ni l'autre ne possède la clé nécessaire au décryptage des données.

- un module de traitement d'un des boitiers électroniques ou du dispositif d'analyse est configuré pour traiter l'ensemble généré de données de façon à segmenter chaque pas en une pluralité de phases, de préférence en au moins quatre phases telles que : une phase d'impact, une phase d'appui, une phase de propulsion et une phase de vol.

- le dispositif d'analyse est configuré :

  o pour comparer les données générées par la plateforme inertielle de chacun des boitiers à des motifs prédéterminés, lesdits motifs prédéterminés correspondants à une catégorie de mouvement prédéterminée,

  o pour générer des valeurs de similarités des données la plateforme inertielle de chacun des boitiers avec les motifs prédéterminés, et

  o pour sélectionner une catégorie de mouvement représentative des données générées sur une période de temps, à partir des valeurs de similarités du premier boitier et celles du second boitier.

Un tel traitement mis en oeuvre par un dispositif d'analyse est rapide et permet une analyse fine de la démarche. En outre, les motifs prédéterminés sont de préférence des motifs prédéterminés à partir de mouvements de calibration réalisés par l'utilisateur. Cela permet une adaptation fine de la caractérisation contrairement à d'autres systèmes déjà développés.

- le dispositif autonome embarqué est configuré de façon à pouvoir s'appairer avec un dispositif comportant une passerelle de connexion à internet.

- les ensembles générés de données transférées au dispositif d'analyse sont des données échantillonnées à au moins 100 Hz.

- le dispositif autonome embarqué comporte une plateforme inertielle.

- le module de communication est configuré pour transmettre les ensembles générés de données réceptionnés de façon indirecte et que le transfert au dispositif d'analyse se fait par l'intermédiaire d'un autre objet connecté.

Selon un autre aspect, l'invention porte sur un procédé de caractérisation de la posture et de la démarche d'un utilisateur mis en œuvre au sein d'un système comportant deux boitiers électroniques intégrés dans une paire de semelles, un premier boitier étant intégré dans une première semelle et un second boitier étant intégré dans une seconde semelle d'une même paire de semelle, chaque boitier comprenant : une plateforme inertielle et un moyen de communication, ledit procédé comprenant :

- Une étape de génération de données, par des plateformes inertielles contenues dans le premier et le second boitier électronique, lesdites données étant générées pour une période de temps et étant fonction de la démarche d'un utilisateur,

- Une étape de transmission de données, par des plateformes inertielles contenues dans le premier et le second boitier électronique, à un dispositif autonome embarqué,

- Une étape de transmission de données, par dispositif autonome embarqué, à un dispositif d'analyse,

- Une étape de traitement des données, par le dispositif d'analyse, comprenant la comparaison des données à des motifs prédéterminés et la génération de valeurs de similarités des données avec les motifs prédéterminés, lesdits motifs prédéterminés correspondants à une catégorie de mouvement prédéterminée, et

- Une étape de sélection, par le dispositif d'analyse, d'une catégorie de mouvement représentative des données générées sur la période de temps, à partir des valeurs de similarités du premier boitier et celles du second boitier électronique.

D'autres avantages et caractéristiques de l'invention apparaitront à la lecture de la description suivante donnée à titre d'exemple illustratif et non limitatif, en référence aux Figures annexées qui représentent :

  [Fig. 1], une vue en coupe longitudinale du dessus de deux semelles chacune contenant une cavité qui va laisser place à un boitier, chacune des antennes des boitiers étant située sur l'arrête extérieure vis à vis de chaque pied, selon un mode de réalisation de l'invention.

  [Fig. 2], un boitier électronique ouvert vu du dessus comprenant notamment une carte électronique, une batterie rechargeable, un connecteur et une antenne.

  [Fig. 3], un boitier électronique, en vue éclatée en coupe de profil, comprenant notamment une batterie rechargeable, une carte électronique ainsi qu'une enveloppe extérieure en deux parties.

  [Fig. 4], un dispositif autonome embarqué selon un mode de réalisation de l'invention.

  [Fig. 5], un schéma représentatif du procédé selon un mode de réalisation de l'invention.

Des aspects de la présente invention sont décrits en référence à des organigrammes et / ou à des schémas fonctionnels de procédés, d'appareils (systèmes) et de produits de programme d'ordinateur selon des modes de réalisation de l'invention.

Sur les figures, les organigrammes et les schémas fonctionnels illustrent l'architecture, la fonctionnalité et le fonctionnement d'implémentations possibles de systèmes, de procédés et de produits de programme d'ordinateur selon divers modes de réalisation de la présente invention. A cet égard, chaque bloc dans les organigrammes ou blocs-diagrammes peut représenter un système, un dispositif, un module ou un code, qui comprend une ou plusieurs instructions exécutables pour mettre en œuvre la ou les fonctions logiques spécifiées. Dans certaines implémentations, les fonctions associées aux blocs peuvent apparaître dans un ordre différent que celui indiqué sur les figures. Par exemple, deux blocs montrés successivement peuvent, en fait, être exécutés sensiblement simultanément, ou les blocs peuvent parfois être exécutés dans l'ordre inverse, en fonction de la fonctionnalité impliquée. Chaque bloc des schémas de principe et / ou de l'organigramme, et des combinaisons de blocs dans les schémas de principe et / ou l'organigramme, peuvent être mis en œuvre par des systèmes matériels spéciaux qui exécutent les fonctions ou actes spécifiés ou effectuer des combinaisons de matériel spécial et d'instructions informatiques.

**[Description de l'invention]**

Dans la suite de la description, on entend par « semelle » un objet permettant de séparer le pied de l'utilisateur du sol. Une chaussure peut comporter une couche de semelle supérieure en contact direct avec le pied de l'utilisateur et une couche de semelle inférieure en contact direct avec le sol ou plus généralement l'environnement extérieur. Une chaussure peut aussi comporter une semelle interne amovible.

Au sens de l'invention, la « démarche » au sens de l'invention correspond à la posture, les mouvements, la locomotion comme la course ou la marche, et l'équilibre de l'utilisateur. L'équilibre correspond notamment à l'équilibre postural lié à la stabilité du corps et plus particulièrement à la stabilité du centre de gravité d'un utilisateur. Néanmoins il peut intégrer aussi bien l'équilibre statique au l'équilibre dynamique.

La « caractérisation de la démarche » correspond, au sens de l'invention, à l'attribution d'une ou de plusieurs valeurs par exemple un score, un classement ou une note à une trajectoire ou au déplacement d'un pied d'un utilisateur. Cette caractérisation de la démarche permet d'obtenir une ou plusieurs valeurs numériques ou alphanumériques de paramètre bio-mécanique représentatifs de la démarche.

Par « paramètre bio-mécanique », une caractéristique de la démarche de l'utilisateur.

Par « modèle » ou « règle » ou « algorithme » il faut comprendre au sens de l'invention une suite finie d'opérations ou d'instructions permettant de calculer une valeur par l'intermédiaire d'un classement ou d'un partitionnement des données au sein de groupes préalablement définis Y et d'attribuer un score ou de hiérarchiser une ou plusieurs données au sein d'un classement. La mise en œuvre de cette suite finie d'opérations permet par exemple d'attribuer une étiquette Y à une observation décrite par un ensemble de caractéristiques ou paramètres X grâce par exemple à la mise en œuvre d'une fonction f susceptible, de reproduire Y ayant observé X.

$$Y = f(X) + e$$

où e symbolise le bruit ou erreur de mesure

Par « pattern », on entend la manière dont l'utilisateur déroule son pied à l'occasion de sa marche ou de sa course.

On entend par « traiter », « calculer », « déterminer », « afficher », « transformer », « extraire » « comparer » ou plus largement « opération exécutable », au sens de l'invention, une action effectuée par un dispositif ou un processeur sauf si le contexte indique autrement. À cet égard, les opérations se rapportent à des actions et/ou des processus d'un système de traitement de données, par exemple un système informatique ou un dispositif informatique électronique, qui manipule et transforme les données représentées en tant que quantités physiques (électroniques) dans les mémoires du système informatique ou d'autres dispositifs de stockage, de transmission ou d'affichage de l'information. Ces opérations peuvent se baser sur des applications ou des logiciels.

Les termes ou expressions « application », « logiciel », « code de programme », et « code exécutable » signifient toute expression, code ou notation, d'un ensemble d'instructions destinées à provoquer un traitement de données pour effectuer une fonction particulière directement ou indirectement (e.g. après une opération de conversion vers un autre code). Les exemples de code de programme peuvent inclure, sans s'y limiter, un sous-programme, une fonction, une application exécutable, un code source, un code objet, une bibliothèque et/ou tout autre séquence d'instructions conçues pour l'exécution sur un système informatique.

On entend par « composite plastique », au sens de l'invention, un matériau multi-composants comprenant au moins deux composants non miscibles dans lequel au moins un composant est un polymère (thermoplastique ou thermodurcissable) et l'autre composant peut être un renfort tel qu'un renfort fibreux.

On entend par « renfort fibreux » ou « substrat fibreux », au sens de l'invention, plusieurs fibres, des stratifils unidirectionnels ou un mat à filament continu, des tissus, des feutres ou des non-tissés qui peuvent être sous la forme de bandes, nappes, tresses, mèches ou pièces.

Par « polymère », on entend soit un copolymère soit un homopolymère. On entend par « copolymère », un polymère regroupant plusieurs unités monomères différentes et par « homopolymère », un polymère regroupant des unités monomères identiques.

On entend par « polymère thermoplastique », au sens de l'invention, un polymère généralement solide à température ambiante, pouvant être cristallin, semi-cristallin ou amorphe, et qui se ramollit lors d'une augmentation de température, en particulier après passage de sa température de transition vitreuse (Tg) et s'écoule à plus haute température. Des exemples de thermoplastiques sont, par exemple : le polyéthylène basse densité (PEHD), le polyéthylène téréphtalate (PET) ou le polychlorure de vinyle (PVC).

On entend par « Polymère thermodurcissable » une matière plastique qui se transforme de manière irréversible par polymérisation en un réseau polymère insoluble.

On entend par « amovible » la capacité à être détachée, enlevée ou démontée aisément sans avoir à détruire des moyens de fixation soit parce qu'il n'y a pas de moyen de fixation soit parce que les moyens de fixation sont aisément et rapidement démontables (e.g. encoche, vis, languette, ergot, clips). Par exemple, par amovible, il faut comprendre que l'objet n'est pas fixé par soudure ou par un autre moyen non prévu pour permettre de détacher l'objet.

Dans cette description et même avant, les mêmes références sont utilisées pour désigner les mêmes éléments.

Les dispositifs ou systèmes existants présentent généralement une pluralité de capteurs (e.g. capteurs de pression) répartis dans la chaussure et/ou semelle. Une telle répartition des capteurs entraine une réduction de la robustesse du système. Au contraire, les systèmes intégrant tous les capteurs dans un seul boitier robuste peuvent souffrir d'une autonomie limitée.

Face à ces insuffisances, l'inventeur a développé un système pour la caractérisation de la démarche d'un utilisateur s'appuyant sur deux boitiers électroniques robustes, dotés de plateforme inertielle générant suffisamment de données brutes pour une analyse fine de la démarche et pouvant communiquer avec un dispositif autonome embarqué capable de réceptionner les données brutes de façon à libérer la mémoire des boitiers électronique et augmenter leur autonomie puis capable d'envoyer ces données à un dispositif d'analyse disposant des capacités de calcul suffisantes pour caractériser la démarche de l'utilisateur.

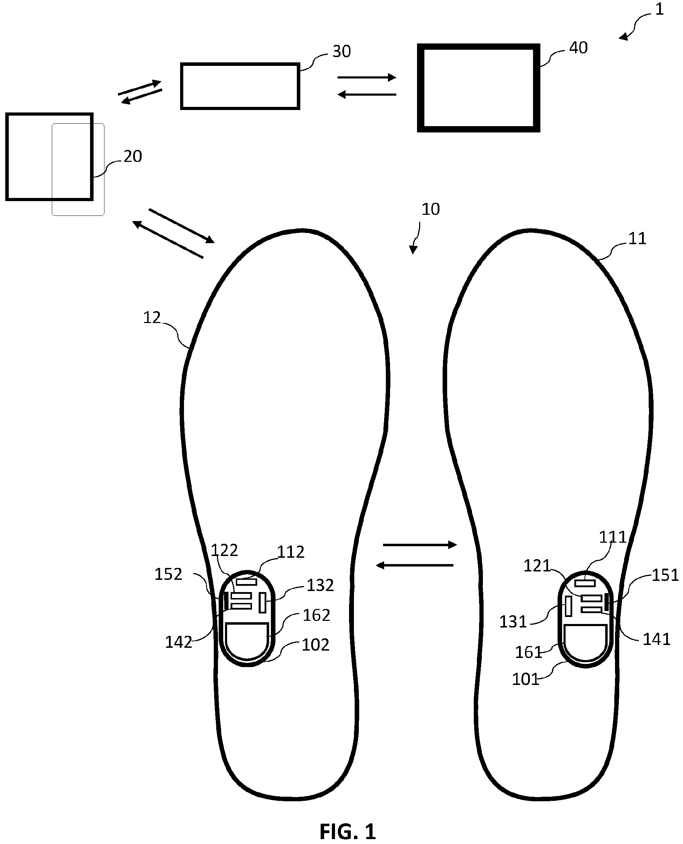

Un tel système 1 pour la caractérisation de la démarche d'un utilisateur est schématisé sur la **Figure 1.**

Pour rappel, les deux pieds contiennent le quart de tous les os du corps humain. Dans chaque pied, il est possible d'identifier 26 os, 33 muscles, 16 articulations et 107 ligaments. Les pieds portent le poids du corps en position debout et permettent la locomotion, assurant un rôle primordial dans l'équilibre, l'amortissement et la propulsion. Les pieds effectuent aussi plusieurs types de mouvements. En outre, les pieds comptent près de 7 200 terminaisons nerveuses, de sorte que toutes les maladies et autres troubles notamment neurologiques sont perceptibles directement ou indirectement au niveau de nos pieds et, d'autre part, peuvent être détectés à partir de notre manière de marcher ou de nous mouvoir.

Pour poursuivre cet objectif, l'invention consiste en une technologie autonome insérée dans un boitier compact et miniaturisé de quelques grammes, qui est inséré dans chacune des deux semelles intérieures et/ou extérieure de chaussures d'une même paire.

Ce sont les choix décrit ci-après qui ont permis aux inventeurs d'obtenir un boitier compact et miniaturisé de quelques grammes qui est néanmoins capable de résister à des pressions et forces répétées dans le cadre de marche, courses sauts ou autres activités sportives.

**Selon un premier aspect,** l'invention porte sur **un système 1 de caractérisation, ou de qualification, de la posture et de la démarche d'un utilisateur.** Un tel système 1 peut aussi être qualifié de système de caractérisation du mouvement des pieds d'un utilisateur.

Comme illustré sur la figure 1, ce système comporte : une paire de boitiers électroniques 101, 102, un dispositif autonome embarqué 20, et un dispositif d'analyse 30. Avantageusement, il peut également comporter un dispositif de visualisation 40 des informations sur la posture et la démarche d'un utilisateur de la paire de semelles.

En particulier, le système 1 selon l'invention peut comporter une paire 10 de semelles comportant les boitiers électroniques 100, 101, 102 selon l'invention et éventuellement un terminal externe 20.

Les semelles 11, 12 utilisables dans le cadre du système 1 selon l'invention peuvent par exemple correspondre à des semelles extérieures ou à des semelles intérieures, de chaussures. Ces semelles peuvent être amovibles ou être intégrées de manière permanente au semelage des chaussures.

Classiquement, les semelles 11, 12 composants ladite paire 10 de semelles, comportent chacune un boitier électronique 100,101,102. Comme présenté sur la figure 1, le boitier électronique 101,102 est de préférence positionné au niveau d'une portion médiane de la semelle.

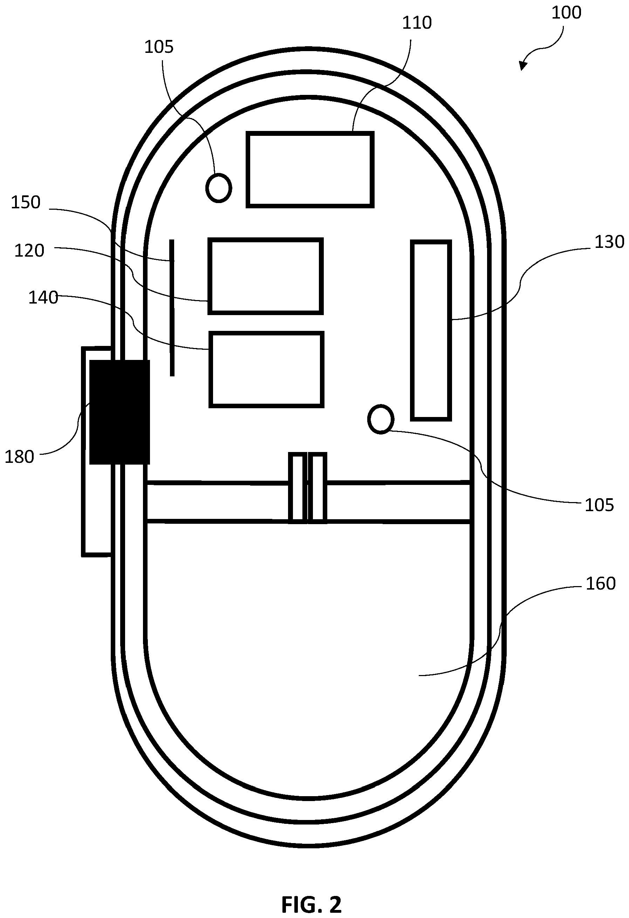

**Un boitier électronique** 100 selon l'invention est illustré à la **figure 2.** Ne pesant que quelques grammes et étant de dimension réduite, ce boitier électronique 100 se loge de façon peu encombrante dans n'importe quelle semelle intérieure et/ou extérieure. Ce faible volume limite l'impact sur le confort de l'utilisateur et présente l'avantage d'optimiser les coûts de production en rendant moins onéreux et plus simple l'intégration de cette technologie dans la semelle lors du processus industriel.

Le choix de la matière de ce boitier électronique est réalisé de façon à assurer sa solidité ainsi que la possibilité de l'insérer dans une semelle. En effet, il convient de pouvoir fabriquer un produit qui puisse d'une part, résister au poids d'une personne et, d'autre part, être facilement inséré dans une semelle ou une chaussure. Allier miniaturisation et résistance du boitier est un véritable challenge : il a fallu réaliser de nombreux prototypes avant de déterminer la matière qui permette d'insérer un tel boitier dans une semelle, sans altérer le confort de celle-ci.

Avantageusement, chaque boitier électronique 100 comporte une enveloppe extérieure 103, ladite enveloppe extérieur étant essentiellement constituée d'un matériau de type composite plastique sélectionné parmi : un matériau composite thermoplastique ou un matériau composite thermodurcissable. De façon préférée, le matériau utilisé est à base de thermoplastique tel que le polycarbonate et peut comprendre du nylon ou de la fibre de verre. En effet, le polycarbonate a l'avantage de pouvoir être thermoformé, résistant mécaniquement et peu inflammable ce qui est avantageux lors de la soudure par ultrason détaillée ci-après.

Le choix d'un matériau en polymère, par exemple en composite plastique, permet d'allier à la fois légèreté, efficacité de la transmission du signal et surtout solidité. Avantageusement, l'enveloppe est arrondie, autrement dit, elle ne possède pas d'angle inférieur à 95°. Cette forme d'enveloppe permet d'améliorer le confort de l'utilisateur.

Ainsi, chaque boitier électronique est de préférence léger et pèse par exemple moins de 20 grammes, moins de 10 grammes, de préférence moins de 8 grammes et de façon plus préférée moins de 6 grammes. En outre, il peut présenter une épaisseur inférieure à 7 mm, ou inférieure à 5 mm, de préférence inférieure à 4 mm et de façon plus préférée inférieure à 3 mm. Cela lui permet de s'intégrer facilement dans une chaussure/semelle sans altérer le confort de l'utilisateur dans sa chaussure. Enfin, chaque boitier électronique présente par exemple moins de 10 cm$^2$ de surface sur sa face la plus grande, de préférence moins de 5 cm$^2$ de surface sur sa face la plus grande, de plus préférée moins de 4 cm$^2$ et de façon encore plus préférée moins de 3 cm$^2$.

De façon préférée, l'enveloppe extérieure 103 du boitier électronique 100 comporte une partie supérieure 103a et une partie inférieure 103b qui sont soudées. Une telle soudure, par exemple une soudure par ultrasons, permet d'augmenter la résistance à l'eau du boitier électronique. Alternativement, la partie supérieure 103a et la partie inférieure 103b peuvent être séparées par un joint en polymère et maintenues par des moyens de fixation amovibles. Ainsi, chaque boitier électronique peut comporter une enveloppe extérieure formée en deux parties et un joint venant se positionner entre deux parties de l'enveloppe extérieure.

De préférence de forme arrondie pour augmenter sa résistance mécanique, il doit être assemblé de telle sorte à maintenir une étanchéité parfaite et rendre l'intérieur contenant la carte électronique et la source d'énergie protégé de l'humidité et des poussières.

Chaque boitier électronique intègre avantageusement des piliers ou plots de soutien 104 afin de renforcer sa solidité, de préférence un plot/cm$^2$ pour résister aux pressions et forces d'impact des mouvements du pied. L'insertion de tels plots permet au boitier de mieux résister au poids d'une personne. De manière avantageuse, ces plots ou encore muret, permettent également de fixer une carte électronique afin de fixer le centre inertiel pour ne pas fausser les mesures. Ainsi, de façon préférée, le boitier électronique 100 selon l'invention comporte au moins deux plots de soutien 104, de façon plus préférée au moins trois plots de soutien 104 et de façon encore plus préférée au moins quatre plots de soutien 104.

En outre, le boitier électronique 100 selon l'invention peut comporter un muret en polymère positionné entre la source d'énergie 160,161,162 et la carte électronique de façon à pouvoir augmenter sa robustesse. Un tel muret présente par exemple une hauteur correspondant à la hauteur du boitier, une épaisseur comprise entre 0,5 et 3 mm et une longueur comprise entre 3/5 et 5/5 de la largeur de la source d'énergie.

Avantageusement, le boitier électronique 100 comporte une carte électronique présentant au moins une ouverture 105 permettant le passage d'au moins un plot de soutien 104, de préférence au moins deux ouvertures 105.

En outre, de façon à augmenter encore la robustesse du système, chaque boitier électronique comporte un matériau absorbeur de chocs tel que de la mousse polymère (e.g. polyuréthane, polyéther). Selon un mode de réalisation, le matériau absorbeur de chocs présente une densité comprise entre 20 kg/m$^3$ et 50 kg/m$^3$. Une telle couche de mousse protectrice permet également d'isoler la carte des vibrations et de l'humidité.

De façon préférée, chacun des boitiers (e.g. droit et gauche) présente une forme différente de l'autre boitier. Par exemple, il comporte une saillie n'étant pas présente sous une forme identique sur l'autre boitier. Une telle caractéristique physique permet de différencier les deux boitiers, droit ou gauche, lors de leur intégration dans une chaussure ou une semelle sans inverser le centre inertiel.

Selon un mode de réalisation de l'invention, la carte électronique est insérée dans un compartiment du boitier spécialement conçu pour le recevoir.

Selon un autre mode de réalisation, le boitier électronique 100 est formé par l'encapsulation de ses composants. Par exemple, l'encapsulation peut prendre la forme un revêtement enrobant ou d'une résine (e.g. silicone, époxy, polyuréthane). L'encapsulation de l'intégralité des composants (e.g. plateforme inertielle, module de traitement...) offre une bonne isolation et combine ainsi de bonnes propriétés électriques à une excellente protection mécanique.

Avantageusement, chaque boitier électronique 100, 101, 102 comporte une enveloppe extérieure 103, ladite enveloppe extérieure étant essentiellement constituée d'un matériau thermoplastique lui permettant de résister à de fortes contraintes mécaniques, correspondant au minimum à 100 000 impacts de 1000 N à une fréquence de 1Hz ou 100 000 impacts de 3000 N à une fréquence de 2,6 Hz, lesdits boitiers étant par ailleurs résistant à la poussière et l'humidité à un niveau au minimum IP56.

En outre, le boitier électronique selon l'invention comporte une plateforme inertielle 110,111,112 configurée pour générer un ensemble de données sur la démarche d'un utilisateur de la paire 10 de semelles. En particulier, la plateforme inertielle 110, 111, 112 est configurée pour générer un ensemble de données sur un mouvement du pied d'un utilisateur de la paire 10 de semelles.

Au cours de la marche d'un utilisateur, la plateforme inertielle 110 acquière des signaux représentatifs d'un paramètre de mouvement (accélération et/ou vitesse, par exemple vitesse angulaire) du pied selon les axes X, Y, Z. En outre, ces données peuvent ensuite être traitées pour générer au moins un signal d'accélération.

Le boitier électronique peut également comporter un ou plusieurs magnétomètres de façon à acquérir trois signaux bruts supplémentaires correspondant aux valeurs de champs magnétiques sur trois dimensions.

Chaque boitier électronique peut comporter par ailleurs d'autres capteurs, notamment un inclinomètre, un baromètre, un capteur de température et un altimètre pour bénéficier d'une précision accrue.

La plateforme inertielle est par exemple constituée d'au moins un accéléromètre et un gyroscope. De façon préférée, elle comporte plusieurs accéléromètres et gyroscopes. De façon plus préférée, la plateforme inertielle 110, 111, 112 comporte au moins un accéléromètre et au moins un gyroscope, et peut être complétée par d'autres capteurs, notamment un magnétomètre, un baromètre et un altimètre.

En outre, avantageusement, les données générées par les boitiers électroniques sont cryptées. Dans ce cas, avantageusement, seul le dispositif d'analyse est apte à décrypter les données générées. Ainsi, le dispositif autonome embarqué ne dispose pas des clés de décryptages.

En particulier, les données générées par les boitiers électroniques sont cryptées à l'aide de clés publiques chacune associée à un des boitiers électroniques et le dispositif d'analyse dispose des clés privées nécessaires au décryptage des données générées. Alternativement, les données générées par les boitiers électroniques sont cryptées à l'aide d'une clé publique associée à plusieurs boitiers électroniques et le dispositif d'analyse dispose de la clé privée nécessaire au décryptage des données générées.

En outre, le boitier électronique selon l'invention peut comporter un module de traitement de données 120,121,122 configuré pour transformer l'ensemble des données générées. Ainsi, les boitiers peuvent être configuré pour réaliser un traitement des signaux générés par la plateforme inertielle de façon à faciliter le traitement ultérieur par le dispositif d'analyse. Le système selon l'invention permet que les données reçues via les capteurs situés dans les semelles intérieures et/ou extérieures sont traitées selon un ou plusieurs algorithmes dans chacun des boîtiers. Le module de traitement est avantageusement configuré pour réaliser un prétraitement des données générées et éventuellement pour réaliser un traitement suffisant pour générer une information sur la posture ou la démarche de l'utilisateur, information que le boîtier transmet au dispositif autonome embarqué en temps réel ou de manière différée avec les données brutes.

Les informations générées peuvent aussi être transmises au second boitier par une émission de signaux qui peuvent être de type Bluetooth.

Lorsqu'un boitier électronique n'est pas en mesure de communiquer en temps réel avec l'autre boitier et/ou avec le dispositif autonome embarqué, il stocke les informations récoltées et les transmettra en différé lorsque l'échange sera de nouveau possible. Cette transmission en différé des données recueillies est rendue possible grâce à la capacité de stockage dont est doté chacun des boitiers électroniques.

Ainsi, le boitier électronique selon l'invention comporte un module de stockage 130,131,132 de données, configuré pour mémoriser au moins une partie des données transformées et/ou des données générées par le module de traitement. Comme cela a déjà été discuté, le dispositif ou système selon l'invention est tel qu'il permet de fonctionner avec un module de stockage de données de faible capacité. Par exemple, le module de stockage 130,131,132 de données peut avantageusement présenter une capacité de mémoire inférieure à 512 ko, de préférence inférieure à 128 ko, de façon plus préférée inférieure à 32 ko et de façon encore plus préférée inférieure à 16 ko. En particulier, le module de stockage peut correspondre à de la mémoire disponible sur un CPU.

En outre, le boitier électronique selon l'invention comporte des moyens de communications. Ainsi, en particulier, chacun des boîtiers, est conçu de manière à pouvoir communiquer indépendamment avec l'autre et/ou directement avec le dispositif autonome embarqué afin de pouvoir échanger ses propres informations sur la posture/le mouvement/l'activité de son pied, dont il a reçu les données via les différents capteurs de sa semelle intérieure et/ou extérieure de chaussure.

De préférence, le boitier électronique selon l'invention comporte un premier moyen de communication 140,141,142 configuré de façon à ce que le boitier électronique 100 d'au moins une des semelles soit apte à transmettre au moins une partie des données brutes à un dispositif autonome embarqué 20. Ces données peuvent être transmises en temps réel ou en différé au dispositif autonome embarqué 20. En particulier, le moyen de communication 140, 141, 142) est configuré de façon à transmettre l'ensemble généré de données à un dispositif autonome embarqué 20. Comme présenté, ces données sont des données dites brutes, c'est-à-dire des données telles que générées par la plateforme inertielle (de préférence 9 axes et au moins à 200 Hz). En particulier, le moyen de communication 140 peut être configuré pour envoyer des données prétraitées afin d'accélérer l'analyse au niveau du dispositif d'analyse. Dans ce cas, la transmission comporte les données prétraitée et les données brutes. Ainsi, il sera toujours possible de retraiter les données brutes pour plus de précision alors que la transmission de données prétraitées permet d'accélérer l'analyse par le dispositif d'analyse 30.

Avantageusement, chaque boitier électronique comporte en outre un second moyen de communication configuré de façon à ce que le boitier électronique 101 d'une première semelle soit apte à communiquer avec le boitier électronique 102 d'une seconde semelle. Ainsi les boitiers électroniques seront capables d'échanger des informations en temps réel. En effet, la génération des données par les plateformes inertielles doivent être synchronisées et cela passe avantageusement par une communication entre les deux boitiers électroniques. De façon plus préférée, les boitiers électroniques sont configurés de façon à pouvoir vérifier ponctuellement leur synchronisation. Par exemple, les boitiers électroniques sont configurés de façon à pouvoir vérifier leur synchronisation à une fréquence inférieure à 10 minutes, de préférence à une fréquence inférieure à 5 minutes, de façon préférée à une fréquence inférieure à 1 minute. En particulier, les deux boitiers électroniques sont configurés pour communiquer entre eux et pour initier la génération de données sur le mouvement du pied d'un utilisateur seulement après réception d'un message du dispositif autonome embarqué 20 et d'un message de l'autre boitiers électronique.

Les premiers et deuxièmes moyens de communication peuvent consister en un seul et même moyen.

Les premiers et deuxièmes moyens de communication sont aptes à recevoir et à transmettre les données sur au moins un réseau de communication. De préférence la communication est opérée par l'intermédiaire d'un protocole sans fils tel que wifi, 3G, 4G, et/ou Bluetooth. De préférence le protocole de communication est un protocole BLE ou ANT+. Ces protocoles de communications permettent une consommation faible en énergie.

Avantageusement, chacun des boitiers 100, 101, 102 est conçu de manière à pouvoir communiquer avec le second boitier et/ou directement avec le dispositif autonome embarqué 20, par exemple par des signaux d'ondes courtes ou hautes fréquences de type Bluetooth Low Energy ou ANT+.

Avantageusement, du fait de son confinement à l'intérieur d'un boitier placé sous le corps d'une personne, l'antenne 150, 151, 152 doit de préférence être disposée à l'intérieur du boitier sur le côté orienté vers le côté extérieur de la semelle. Ce positionnement de l'antenne est préférable dans la mesure où des tests en laboratoire ont permis d'établir que le signal émis depuis une semelle ou une chaussure est absorbée à 70 % par le corps humain. Cette antenne doit donc être positionnée en périphérie du pied et orientée de telle manière à toujours pouvoir transmettre le signal au dispositif autonome embarqué 20 et/ou à au boitier de la seconde semelle. De façon préférée, l'antenne peut être une antenne imprimée sur une carte électronique. Alternativement, l'antenne peut être imprimée sur une face intérieure du boitier et connectée à la carte électronique par câblage. L'antenne peut de préférence être positionnée sur une partie basse par rapport à la carte électronique. Ainsi, la carte électronique vient faire contact avec l'antenne.

En outre, le boitier électronique selon l'invention comporte une source d'énergie 160,161,162. La source d'énergie est de préférence de type batterie, rechargeable ou non. De préférence la source d'énergie est une batterie rechargeable. En outre, elle peut être associée à un système de recharge par le mouvement ou par une énergie extérieure. Le système de recharge par une énergie extérieure peut notamment être un système de recharge par connexion filaire, un système de recharge par induction ou encore photovoltaïque.

Le boitier électronique 100, 101, 102 peut comporter une source d'énergie 160 de type batterie rechargeable, dont la recharge peut être réalisée selon différentes technologies :

par chargeur, avec un connecteur affleurant au niveau de la semelle ;

avec un dispositif de recharge mécanique intégré à la semelle, comme par exemple un dispositif piézoélectrique apte à fournir une énergie électrique à partir de la marche ;

avec un dispositif sans contact, par exemple par induction ; ou

avec un dispositif photovoltaïque.

En outre, le boitier électronique selon l'invention peut comporter un moyen de connexion filaire 180, de préférence protégé par une languette amovible. Ce moyen de connexion filaire peut être par exemple un port USB ou firewire. Avantageusement, le port USB est également résistant à l'eau ou l'humidité. En outre, le port USB est avantageusement surmonté d'une poutrelle en polymère permettant de lui conférer une plus grande résistance en condition d'utilisation. Ce moyen de connexion filaire peut être utilisé comme évoqué ci-dessus pour recharger la batterie mais également pour échanger des données et par exemple mettre à jour le micrologiciel de la carte électronique portant les différents composants du boitier électronique.

De préférence, la languette amovible ou USB cover, permet de protéger le port USB de corps étrangers. Par exemple, la languette amovible permet de protéger le port USB de l'eau ou de la poussière. Une telle languette peut de préférence être réalisée en polymère de type élastomère ou polyuréthane.

Ces différents composants du boitier électronique sont de préférence agencés sur une carte électronique 170 (ou circuit imprimé). En outre, les différents moyens et modules du boitier électronique 100 sont représentés de façon distincte sur les figures 1 et 2 mais l'invention peut prévoir divers types d'agencement comme par exemple un seul module cumulant l'ensemble des fonctions décrites ici. De même, ces moyens peuvent être divisés en plusieurs cartes électroniques ou bien rassemblés sur une seule carte électronique.

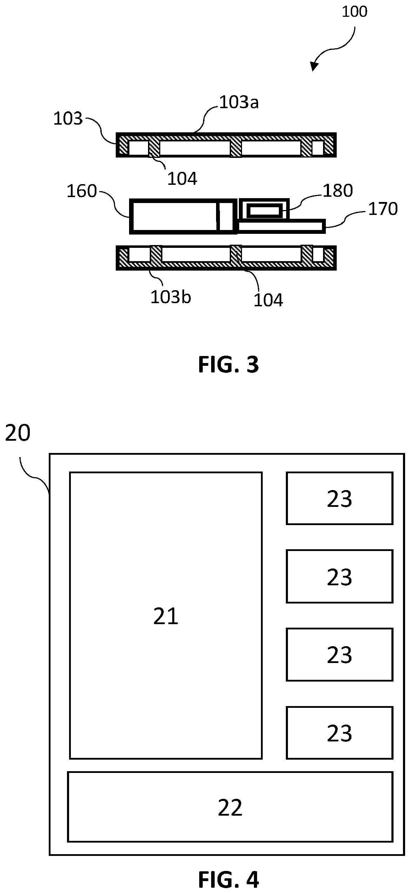

Le système comporte en outre **un dispositif autonome embarqué** 20 illustré à la **figure 4.**

Ce dispositif autonome embarqué 20 comporte notamment un module de communication 21 et un module de stockage de données 22.

Le module de communication 21 configuré pour recevoir les ensembles générés de données transmis par les deux boitiers électroniques et pour transmettre les ensembles générés de données réceptionnés à un dispositif d'analyse 30. Par exemple, le dispositif autonome embarqué 20 communique avec les boitiers électroniques selon un protocole de communication BLE (Bluetooth Low Energy en terminologie anglosaxonne). Le module de stockage de données 22 est configuré pour mémoriser les ensembles générés de données réceptionnés.

Le dispositif autonome embarqué 20 permet de décharger régulièrement les boitiers électroniques des données générées (données brutes) de façon à ne pas saturer leur mémoire et à renforcer l'autonomie des boitiers. Le dispositif autonome embarqué 20 permet de gérer au minimum deux boitiers électroniques 101, 102. Néanmoins, le dispositif autonome embarqué 20 peut aussi être avantageusement configuré pour recevoir et retransmettre les ensembles de données générées par plusieurs paires de boitiers électroniques. En particulier, il peut être configuré pour recevoir et retransmettre les ensembles de données générées par au moins 5 pairs de boitiers électroniques, de préférence au moins 5 pairs de boitiers électroniques.

Pour cela, le dispositif autonome embarqué 20 dispose des identifiants et codes nécessaires pour établir une communication avec chacune des paires de boitiers électronique concernés.

En outre, le dispositif autonome embarqué 20 peut être configuré de façon à pouvoir s'apparier avec un dispositif, tel qu'un modem, un téléphone ou un ordinateur, comportant une passerelle de connexion à internet.

En outre, le dispositif autonome embarqué 20 peut comporter une plateforme inertielle. Lorsque le dispositif autonome embarqué 20 est associé à une seule paire de boitiers électronique alors cela permet d'obtenir une information supplémentaire sur la posture et la démarche de l'utilisateur. De même, le dispositif autonome embarqué 20 comporte au moins un capteur sélectionné parmi : GPS, cardiofréquencemètre, ou anémomètre.

Le module de communication 21 peut être configuré pour transmettre les ensembles générés de données réceptionnés de façon indirecte et que le transfert au dispositif d'analyse 30 se fait par l'intermédiaire d'un autre objet connecté. Par exemple, le transfert peut être réalisé par l'intermédiaire d'un ordinateur auquel le dispositif autonome embarqué est connecté.

En outre, la communication du dispositif autonome embarqué 20 peut se faire par des moyens divers tels qu'un module LoRa. Le dispositif autonome embarqué 20 peut aussi comporter une carte SIM et de préférence son module de communication 21 est configuré pour transmettre les ensembles générés de données réceptionnés selon un protocole de communication sélectionné parmi : 2G, 3G, 4G, ou 5G.

La transmission des ensembles de données générées, de préférence cryptées par le boitier électronique, peut se faire par diverse protocole au dispositif d'analyse. Par exemple, le module de communication 21 du dispositif autonome embarqué 20 peut être configuré pour transmettre les ensembles générés de données réceptionnés selon un protocole de messagerie MQTT (MQ Telemetry Transport en terminologie anglosaxonne).

Comme cela a été évoqué, le dispositif autonome embarqué 20 sert d'intermédiaire entre une ou plusieurs paires de boitiers électroniques et le dispositif d'analyse. Ainsi, avantageusement, il comporte suffisamment d'espace de stockage de données pour enregistrer plusieurs heures de mesures de plusieurs paires de boitiers électronique. En particulier, le dispositif autonome embarqué 20 comporte un module de stockage de donnée apte à mémoriser au moins 2 Go de données, de préférence au moins 4 Go de données, de façon plus préférée au moins 10 Go de données, et de façon encore plus préférée au moins 20 Go de données.

En outre, il peut comporter plusieurs ports 23 dédiés à la communication avec les boitiers électroniques et/ou à leur rechargement. Comme illustré sur la figure 4, le dispositif autonome embarqué 20 comporte avantageusement au moins quatre ports 23 dédiés à la communication avec les boitiers électroniques et/ou à leur rechargement tel que des ports USB ou USB-C.

**Le dispositif d'analyse** 30 configuré pour traiter l'ensemble de données recueillies en fonction d'algorithmes prédéfinis et générer une information sur la posture et la démarche d'un utilisateur de la paire de semelles.

**Le dispositif d'analyse** 30 peut être tout dispositif capable de réaliser des calculs de transformation de données. De préférence, le **dispositif d'analyse** 30 est un ordinateur ou un serveur et de façon plus préférée, le **dispositif d'analyse** 30 est un serveur.

Les données brutes transférées au dispositif d'analyse sont de préférence des données échantillonnées à au moins 100 Hz, de façon plus préférée à au moins 200 Hz, et de façon encore plus préférée à au moins 500 Hz.

Le dispositif d'analyse 30 comporte un module de traitement de données configurée pour transformer l'ensemble des données générées grâce à des algorithmes prédéfinis. En effet, le système selon l'invention permet que les données reçues via les capteurs situés dans les semelles intérieures et/ou extérieures sont traitées selon un ou plusieurs algorithmes de façon à générer une information sur la posture ou la démarche de l'utilisateur.

Le module de traitement permet d'analyser en 3D la posture, les mouvements, la locomotion, l'équilibre et l'environnement de l'utilisateur, et plus généralement tout ce qui sera qualifié comme étant sa marche, à partir des données recueillies par la plateforme inertielle et les éventuels capteurs complémentaires placés dans la semelle.

Ce module de traitement peut être utilisé pour générer des paramètres biomécaniques de la démarche. Avantageusement, le module de traitement de données est apte à transformer l'ensemble de données en au moins un paramètre biomécanique de la démarche, ledit paramètre biomécanique de la démarche étant de préférence sélectionné parmi : la posture, la pronation, la supination, la force d'impact, la zone d'impact, la longueur des pas, le temps de contact, le temps de vol, la boiterie, la force de propulsion, l'équilibre et plusieurs autres paramètres relatifs à l'utilisateur et décrivant sa démarche, ses postures et ses mouvements.

En outre, la transformation par le module de traitement de données peut avantageusement comprendre la segmentation des données en une pluralité de phases. De préférence, le module de traitement de données est apte à segmenter un pas en au moins quatre phases telles que : la phase d'impact (correspond au moment précis du contact du pied avec le sol), la phase d'appui (se déroule depuis la phase d'impact jusqu'au décollement du talon du sol), la phase de propulsion (débute quand le talon a quitté le sol et se termine quand le premier orteil a quitté le sol) et la phase de vol (débute quand le premier orteil a quitté le sol et se termine quand le talon touche le sol).

De façon plus particulière, le découpage ou la segmentation du pas peut permettre d'identifier les zones principales d'appui de l'utilisateur. Ainsi, le système peut être utilisé pour mesurer la forme du pas pendant la marche ou toute autre activité de l'utilisateur afin de déterminer les malformations éventuelles des pieds et postures de l'utilisateur.

En outre, de façon avantageuse, le dispositif d'analyse 30 est configuré :

- pour comparer les données générées par la plateforme inertielle de chacun des boitiers à des motifs prédéterminés, lesdits motifs prédéterminés correspondants à une catégorie de mouvement prédéterminée,

- pour générer des valeurs de similarités des données la plateforme inertielle de chacun des boitiers avec les motifs prédéterminés, et

- pour sélectionner une catégorie de mouvement représentative des données générées sur une période de temps, à partir des valeurs de similarités du premier boitier et celles du second boitier.

Les motifs prédéterminés sont de préférence des motifs prédéterminés à partir de mouvements de calibration réalisés par l'utilisateur. Ainsi, il y a une parfaite adéquation entre les motifs et les habitudes de l'utilisateur. Les mouvements de calibration sont par exemple des étapes de marche ou de montée d'escalier.

**Selon un autre aspect,** l'invention porte sur un **procédé 200 de caractérisation de la démarche** d'un utilisateur, de préférence mis en œuvre au sein d'un système selon l'invention.

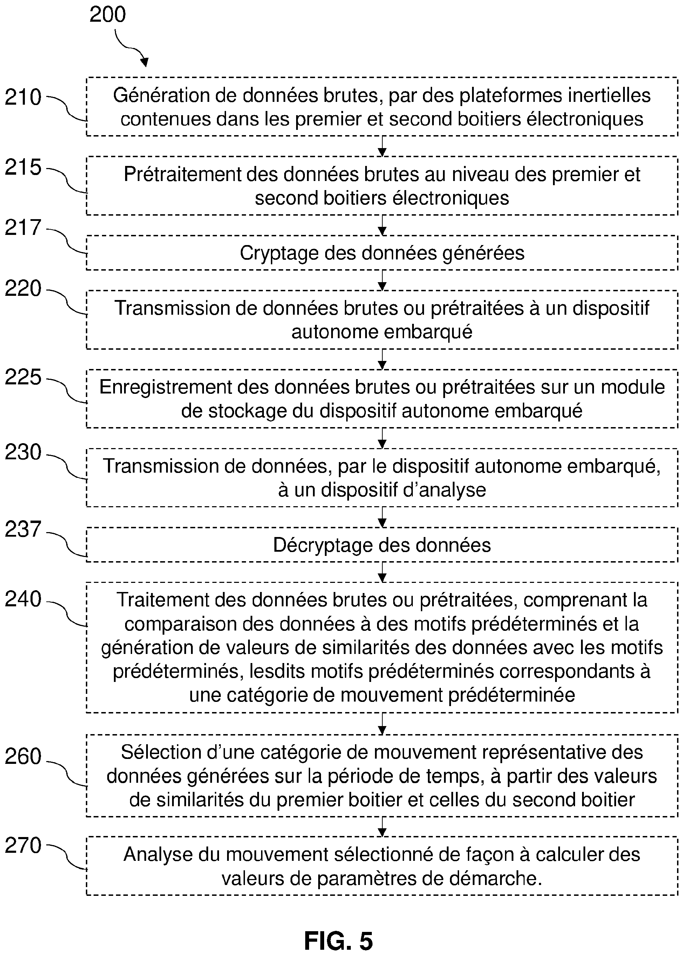

Comme cela est illustré à la **figure 5**, le procédé 200 de caractérisation de la posture et de la démarche d'un utilisateur comprend :

- Une étape de génération 210 de données brutes, par des plateformes inertielles contenues dans les premier et second boitiers électronique, lesdites données brutes étant générées pour une période de temps et étant fonction de la démarche d'un utilisateur,

- Une éventuelle étape 215 de prétraitement des données brutes au niveau des premier et second boitiers électroniques,

- Une étape de transmission 220 de données brutes ou prétraitées, par un module de communication des boitiers électroniques, à un dispositif autonome embarqué 20,

- Une étape d'enregistrement 225 des données brutes ou prétraitées sur un module de stockage du dispositif autonome embarqué,

- Une étape de transmission 230 de données brutes ou prétraitées, par le dispositif autonome embarqué 20, à un dispositif d'analyse 30,

- Une étape de traitement 240 des données brutes ou prétraitées, par le dispositif d'analyse, comprenant la comparaison des données brutes ou prétraitées à des motifs prédéterminés et la génération de valeurs de similarités des données brutes ou prétraitées avec les motifs prédéterminés, lesdits motifs prédéterminés correspondants à une catégorie de mouvement prédéterminée, et

- Une étape de sélection 260, par le dispositif d'analyse, d'une catégorie de mouvement représentative des données brutes ou prétraitées générées sur la période de temps, à partir des valeurs de similarités du premier boitier et celles du second boitier.

En outre, après l'étape de génération 210, le procédé selon l'invention peut comporter une étape de cryptage 217 des données générées. Dans ce cas, il comporte une étape de décryptage 237 des données avant l'étape de traitement 240 desdites données.

En outre, une fois la catégorie de mouvement sélectionnée, le procédé selon l'invention peut comporter une étape 270 d'analyse dudit mouvement de façon à calculer des valeurs de paramètres de démarche.

**Revendications**

1. Système (1) de caractérisation de la posture et de la démarche d'un utilisateur, **caractérisé en ce qu'**il comporte :

   - Une paire de boitiers électroniques (100, 101, 102), aptes à être intégrés dans une paire (10) de semelles, un premier boitier étant apte à être intégré dans une première semelle et un second boitier étant apte à être intégré dans une seconde semelle, chaque boitier comprenant :

      o une plateforme inertielle (110, 111, 112) configurée pour générer un ensemble de données sur un mouvement du pied d'un utilisateur de la paire (10) de semelles, et
      o un moyen de communication (140, 141, 142) configuré de façon à transmettre l'ensemble généré de données à un dispositif autonome embarqué (20),

   - Un dispositif autonome embarqué (20) comprenant :

      o un module de communication (21) configuré pour recevoir les ensembles générés de données transmis par les deux boitiers électroniques, et
      ∘ un module de stockage de données (22) configuré pour mémoriser les ensembles générés de données réceptionnés, le module de communication (21) étant en outre configuré pour transmettre les ensembles générés de données réceptionnés à un dispositif d'analyse (30) ; et

   - un dispositif d'analyse (30) configuré pour traiter les ensembles de données recueillies en fonction d'algorithmes prédéfinis et générer une information sur la posture et la démarche d'un utilisateur de la paire de semelles.

2. Système selon la revendication 1, **caractérisé en ce que** les deux boitiers électroniques sont configurés pour communiquer entre eux et pour initier la génération de données sur le mouvement du pied d'un utilisateur seulement après réception d'un message du dispositif autonome embarqué (20) et d'un message de l'autre boitier électronique.

3. Système selon la revendication 2, **caractérisé en ce que** les boitiers électroniques sont configurés de façon à pouvoir vérifier ponctuellement leur synchronisation, de préférence pour vérifier leur synchronisation à une fréquence inférieure à 10 minutes.

4. Système selon l'une quelconque des revendications précédentes, **caractérisé en ce qu'**il comporte au moins deux paires de boitiers électroniques (100, 101, 102), chacune configurée pour communiquer leurs ensembles générés de données au dispositif autonome embarqué (20).

5. Système selon la revendication 4, **caractérisé en ce que** le dispositif autonome embarqué (20) est configuré pour recevoir et retransmettre les ensembles de données générées par plusieurs paires de boitiers électroniques.

6. Système selon la revendication 5, **caractérisé en ce que** le dispositif autonome embarqué (20) dispose d'identifiants et de codes nécessaires pour établir une communication avec chacune des paires de boitiers électroniques concernés.

7. Système selon l'une quelconque des revendications précédentes, **caractérisé en ce que** les ensembles générés de données sont cryptés par les boitiers électroniques et **en ce que** seul le dispositif d'analyse est apte à décrypter les données générées.

8. Système selon l'une quelconque des revendications précédentes, **caractérisé en ce qu'**un module de traitement d'un des boitiers électroniques ou du dispositif d'analyse (30) est configuré pour traiter l'ensemble généré de données de façon à segmenter chaque pas en une pluralité de phases, de préférence en au moins quatre phases telles que : une phase d'impact, une phase d'appui, une phase de propulsion et une phase de vol.

9. Système selon l'une quelconque des revendications précédentes, **caractérisé en ce que** le dispositif d'analyse (30) est configuré :

   - pour comparer les données générées par la plateforme inertielle de chacun des boitiers à des motifs prédéterminés, lesdits motifs prédéterminés correspondants à une catégorie de mouvement prédéterminée,

- pour générer des valeurs de similarités des données la plateforme inertielle de chacun des boitiers avec les motifs prédéterminés, et

- pour sélectionner une catégorie de mouvement représentative des données générées sur une période de temps, à partir des valeurs de similarités du premier boitier et celles du second boitier.

10. Système selon l'une quelconque des revendications précédentes, **caractérisé en ce que** les motifs prédéterminés sont des motifs prédéterminés à partir de mouvements de calibration réalisés par l'utilisateur.

11. Système selon l'une quelconque des revendications précédentes, **caractérisé en ce que** le dispositif autonome embarqué (20) est configuré de façon à pouvoir s'appairer avec un dispositif comportant une passerelle de connexion à internet.

12. Système selon l'une quelconque des revendications précédentes, **caractérisé en ce que** les ensembles générés de données transférées au dispositif d'analyse sont des données échantillonnées à au moins 100 Hz.

13. Système selon l'une quelconque des revendications précédentes, **caractérisé en ce que** le dispositif autonome embarqué (20) comporte une plateforme inertielle.

14. Système selon l'une quelconque des revendications précédentes, **caractérisé en ce que** le module de communication (21) est configuré pour transmettre les ensembles générés de données réceptionnés de façon indirecte et que le transfert au dispositif d'analyse (30) se fait par l'intermédiaire d'un autre objet connecté.

15. Procédé (200) de caractérisation de la posture et de la démarche d'un utilisateur mis en œuvre au sein d'un système comportant deux boitiers électroniques (100, 101, 102) intégrés dans une paire (10) de semelles, un premier boitier (101) étant intégré dans une première semelle et un second boitier (102) étant intégré dans une seconde semelle d'une même paire de semelle, chaque boitier comprenant : une plateforme inertielle et un moyen de communication, ledit procédé comprenant :

- Une étape de génération (210) de données, par des plateformes inertielles contenues dans le premier et le second boitier électronique, lesdites données étant générées pour une période de temps et étant fonction de la démarche d'un utilisateur,
- Une étape de transmission (220) de données, par des plateformes inertielles contenues dans le premier et le second boitier électronique, à un dispositif autonome embarqué (20),
- Une étape de transmission (230) de données, par dispositif autonome embarqué (20), à un dispositif d'analyse (30),
- Une étape de traitement (240) des données, par le dispositif d'analyse, comprenant la comparaison des données à des motifs prédéterminés et la génération de valeurs de similarités des données avec les motifs prédéterminés, lesdits motifs prédéterminés correspondants à une catégorie de mouvement prédéterminée, et
- Une étape de sélection (260), par le dispositif d'analyse, d'une catégorie de mouvement représentative des données générées sur la période de temps, à partir des valeurs de similarités du premier boitier et celles du second boitier électronique.

EP 3 714 773 A1

**FIG. 1**

**FIG. 2**

**FIG. 3**

**FIG. 4**

200

210 — Génération de données brutes, par des plateformes inertielles contenues dans les premier et second boitiers électroniques

215 — Prétraitement des données brutes au niveau des premier et second boitiers électroniques

217 — Cryptage des données générées

220 — Transmission de données brutes ou prétraitées à un dispositif autonome embarqué

225 — Enregistrement des données brutes ou prétraitées sur un module de stockage du dispositif autonome embarqué

230 — Transmission de données, par le dispositif autonome embarqué, à un dispositif d'analyse

237 — Décryptage des données

240 — Traitement des données brutes ou prétraitées, comprenant la comparaison des données à des motifs prédéterminés et la génération de valeurs de similarités des données avec les motifs prédéterminés, lesdits motifs prédéterminés correspondants à une catégorie de mouvement prédéterminée

260 — Sélection d'une catégorie de mouvement représentative des données générées sur la période de temps, à partir des valeurs de similarités du premier boitier et celles du second boitier

270 — Analyse du mouvement sélectionné de façon à calculer des valeurs de paramètres de démarche.

**FIG. 5**

**RAPPORT DE RECHERCHE EUROPEENNE**

Europäisches Patentamt
European Patent Office
Office européen des brevets

Numéro de la demande
EP 20 16 6526

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2015/257679 A1 (ROSS JOHNNY [US]) 17 septembre 2015 (2015-09-17) * figures 1, 6, 13 * * alinéa [0071] - alinéa [0078] * * alinéa [0082] * * alinéa [0105] - alinéa [0115] * * alinéa [0143] * * alinéa [0154] - alinéa [0155] * * alinéa [0181] * * alinéa [0196] - alinéa [0203] * ----- | 1-15 | INV. A61B5/00 A43B5/00 A61B5/11 ADD. A61B5/01 A61B5/103 |
| X | WO 2010/096691 A2 (UNIV COLORADO REGENTS [US]; SAZONOV EDUARD [US] ET AL.) 26 août 2010 (2010-08-26) * figure 1A * * alinéa [0026] - alinéa [0031] * * alinéa [0073] - alinéa [0083] * * alinéa [0088] - alinéa [0089] * * alinéa [0126] - alinéa [0129] * ----- | 1-15 | |
| X | US 2016/324445 A1 (KIM JEONG-BIN [KR] ET AL) 10 novembre 2016 (2016-11-10) * figures 1, 4, 5, 9, 10A, 10B * * alinéa [0113] - alinéa [0119] * * alinéa [0139] * * alinéa [0155] - alinéa [0160] * ----- | 1-15 | DOMAINES TECHNIQUES RECHERCHES (IPC) A61B B29D A43B H04W |
| A | US 2018/049670 A1 (MARKISON TIMOTHY W [US] ET AL) 22 février 2018 (2018-02-22) * figure 3 * * alinéa [0060] * * alinéa [0092] * ----- | 2,4 | |
| A | WO 2011/003181 A1 (AUTONOMOUS IDENTITY MAN SYSTEMS INC AIMS [CA]; GRAY TODD D [CA] ET AL.) 13 janvier 2011 (2011-01-13) * alinéa [0061] - alinéa [0062] * ----- -/-- | 4 | |

Le présent rapport a été établi pour toutes les revendications

| Lieu de la recherche | Date d'achèvement de la recherche | Examinateur |
|---|---|---|
| La Haye | 19 juin 2020 | Knoop, Jan |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

page 1 de 2

Europäisches
Patentamt

European
Patent Office

Office européen
des brevets

**RAPPORT DE RECHERCHE EUROPEENNE**

Numéro de la demande

EP 20 16 6526

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | Digitsole: "Application Run Profiler : General presentation", Youtube, 22 mars 2017 (2017-03-22), page 1 pp., XP054979904, Extrait de l'Internet: URL:https://www.youtube.com/watch?v=hy5HRGV0usU [extrait le 2019-11-12] * 0:00-1:52 * | 1-15 | |
| | ----- | | |
| A | Digitsole: "Digitsole Run Profiler", Youtube, 21 septembre 2016 (2016-09-21), page 1 pp., XP054979903, Extrait de l'Internet: URL:https://www.youtube.com/watch?v=DWXSS4W5m0 [extrait le 2019-11-12] * 0:00-1:42 * | 1-15 | |
| | ----- | | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| La Haye | 19 juin 2020 | Knoop, Jan |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
 
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 20 16 6526

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

19-06-2020

| Document brevet cité au rapport de recherche | | | Date de publication | Membre(s) de la famille de brevet(s) | | | Date de publication |
|---|---|---|---|---|---|---|---|
| US | 2015257679 | A1 | 17-09-2015 | AUCUN | | | |
| WO | 2010096691 | A2 | 26-08-2010 | EP | 2398383 | A2 | 28-12-2011 |
| | | | | US | 2011054359 | A1 | 03-03-2011 |
| | | | | WO | 2010096691 | A2 | 26-08-2010 |
| US | 2016324445 | A1 | 10-11-2016 | US | 2016324445 | A1 | 10-11-2016 |
| | | | | WO | 2016178523 | A1 | 10-11-2016 |
| US | 2018049670 | A1 | 22-02-2018 | US | 2018049670 | A1 | 22-02-2018 |
| | | | | US | 2018049671 | A1 | 22-02-2018 |
| | | | | US | 2018049703 | A1 | 22-02-2018 |
| | | | | US | 2018052228 | A1 | 22-02-2018 |
| | | | | US | 2018054663 | A1 | 22-02-2018 |
| WO | 2011003181 | A1 | 13-01-2011 | CA | 2791403 | A1 | 13-01-2011 |
| | | | | US | 2013200996 | A1 | 08-08-2013 |
| | | | | WO | 2011003181 | A1 | 13-01-2011 |

EPO FORM P0460

Pour tout renseignement concernant cette annexe : voir Journal Officiel de l'Office européen des brevets, No.12/82

## RÉFÉRENCES CITÉES DANS LA DESCRIPTION

**Documents brevets cités dans la description**

- EP 1970671 A
- WO 2011157870 A
- US 20170241797 A
- US 20170188950 A
- US 2017303827 A